# EUROPEAN PATENT APPLICATION

(11) **EP 4 834 782 A1**
(43) Date of publication of application: **04.11.2026**
(21) Application number: 24912800.0
(22) Date of filing: 24.12.2024
(51) Int. Cl.: B65B 55/08, A61L 2/10, B65B 55/04, B65B 55/10

(54) **CONTAINER STERILIZATION METHOD AND CONTAINER STERILIZATION DEVICE**

(30) Priority: 28.12.2023 JP 2023222772
(71) Applicant: Toyo Seikan Co., Ltd., Tokyo 141-8640 (JP)
(72) Inventor: IWASHITA, Takeshi, Yokohama-shi, Kanagawa 230-0001 (JP)
(74) Representative: Pestalozzi, Deborah
(86) International application number: PCT/JP2024/045667
(87) International publication number: WO 2025/142913

(57) **Abstract**

Provided are a container sterilization method and a container sterilization device for achieving effective sterilization in a short time while avoiding or reducing the risk of remaining sterilizing agent. A container sterilization method for sterilizing a container surface of a container or a container preform, the container sterilization method including: a laser irradiation process of irradiating the container surface with a C0₂ laser; and a UV light irradiation process of irradiating the container surface with UV light.

## Description

### Technical Field

The present invention relates to a container sterilization method and a container sterilization device for sterilizing a container surface of a container or a container preform.

### Background Art

In the related art, as a container sterilization method for sterilizing a container surface of a container or a container preform, a sterilization process using a sterilizing agent such as hydrogen peroxide has been known (for example, see Patent Document 1).

### Citation List

### Patent Document

Patent Document 1: JP 2010-202284 A

### Summary of Invention

### Technical Problem

However, in the sterilization process using the sterilizing agent, the sterilizing agent may remain in the molded container, and thus it is necessary to take measures to reduce the risk of residue.

Here, it is an object of the present invention to solve these problems, and to provide a container sterilization method and a container sterilization device for, with a simple configuration, achieving effective sterilization in a short time while avoiding or reducing the risk of remaining sterilizing agent.

### Solution to Problem

An aspect of a container sterilization method of the present invention is a container sterilization method for sterilizing a container surface of a container or a container preform, the container sterilization method including: a laser irradiation process of irradiating the container surface with a CO₂ laser; and a UV light irradiation process of irradiating the container surface with UV light, thereby solving the above problems.

Further, another aspect of the container sterilization method of the present invention is a container sterilization method for sterilizing a container surface of a container or a container preform, the container sterilization method including: a laser irradiation process of irradiating the container surface with a laser having an infrared wavelength; and a UV light irradiation process of irradiating the container surface with UV light, thereby solving the above problems.

Further, an aspect of a container sterilization device of the present invention is a container sterilization device for sterilizing a container surface of a container or a container preform, including: a laser irradiation means that irradiates the container surface with a CO₂ laser; and a UV light irradiation means that irradiates the container surface with UV light, thereby solving the above problems.

Further, another aspect of the container sterilization device of the present invention is a container sterilization device for sterilizing a container surface of a container or a container preform, including: a laser irradiation means that irradiates the container surface with a laser having an infrared wavelength; and a UV light irradiation means that irradiates the container surface with UV light, thereby solving the above problems.

In the container sterilization method or the container sterilization device, an irradiated region of the CO₂ laser in the laser irradiation process and an irradiated region of the UV light in the UV light irradiation process may overlap each other.

In the container sterilization method or the container sterilization device, in the UV light irradiation process, a UV laser may be used for irradiation.

In the container sterilization method or the container sterilization device, the laser irradiation process and the UV light irradiation process may be performed by mixing the CO₂ laser and the UV laser and emitting the mixed laser from an identical irradiation unit.

In the container sterilization method or the container sterilization device, a sterilization capability against mold by the laser irradiation process may be set to be higher than a sterilization capability against mold by the UV light irradiation process.

In the container sterilization method or the container sterilization device, a sterilization capability against spore-forming bacteria by the laser irradiation process may be set to be lower than a sterilization capability against spore-forming bacteria by the UV light irradiation process.

The container sterilization method or the container sterilization device may further include: a heating process to be performed before or simultaneously with the laser irradiation process, and in the heating process, a region to be sterilized on the container surface by the laser irradiation process may be heated.

The container sterilization method or the container sterilization device may further include: a heating process of heating a preform as the container preform to a molding temperature at which the preform is molded into a PET bottle as the container, the laser irradiation process and the UV light irradiation process may be performed with respect to the preform after the heating process, and an integrated light amount (mJ/cm²) of the CO₂ laser to a mouth portion of the preform may be greater than an integrated light amount (mJ/cm²) of the CO₂ laser to a body portion of the preform, and/or an integrated light amount (mJ/cm²) of the UV light to the mouth portion of the preform may be greater than an integrated light amount (mJ/cm²) of the UV light to the body portion of the preform.

### Advantageous Effects of Invention

According to the present invention, effective sterilization can be realized in a short time with a simple configuration while avoiding or reducing the risk of remaining sterilizing agent.

### Brief Description of Drawings

FIG. 1 is an explanatory diagram illustrating an aseptic filling system including a container sterilization device according to an embodiment of the present invention.
FIG. 2 is an explanatory diagram illustrating an example of an irradiation mode of a CO₂ laser and UV light.
FIG. 3 is an explanatory diagram illustrating results of a test for confirming the sterilization effect of CO₂ laser irradiation.
FIG. 4 is an explanatory diagram illustrating the sterilization effect by UV light irradiation.
FIG. 5 is a diagram illustrating absorbances of spore-forming bacteria, mold, and PET resin with respect to the light wavelength.

### Description of Embodiments

An aseptic filling system 10 according to an embodiment of the present invention will be described below with reference to the drawings.

The terms "upstream" and "downstream" used in the present specification mean upstream and downstream in the conveying direction of a preform P or a container.

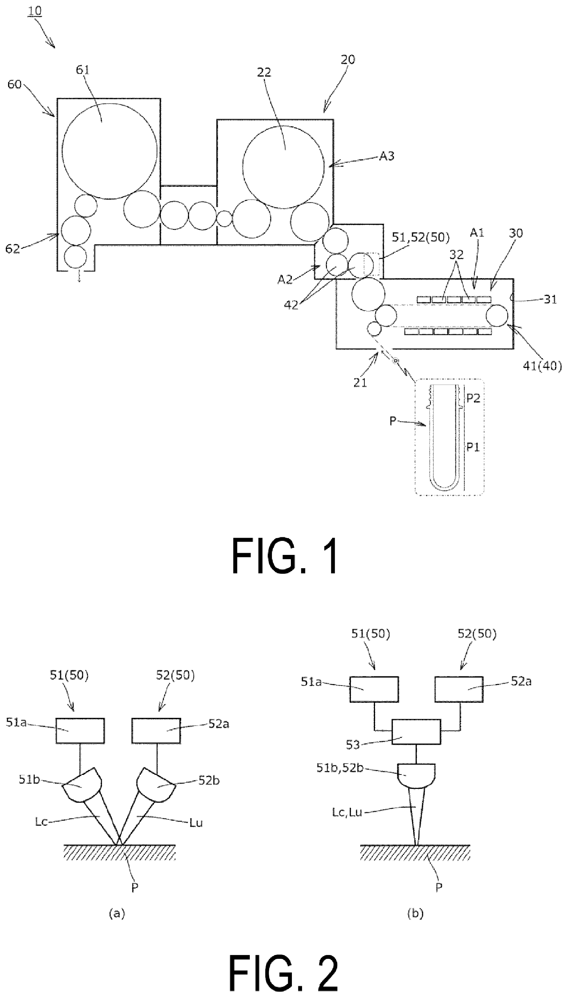

First, the aseptic filling system 10 is configured as a so-called in-line blow type filling system that aseptically fills a sterilized container with a sterilized content (particularly liquid contents), and as illustrated in FIG. 1, includes an aseptic molding device 20 that aseptically molds the container, and a filling device 60 that aseptically fills the container with the content.

Each of the constituent elements of the aseptic filling system 10 will be described below with reference to the drawings.

First, the aseptic molding device 20 aseptically molds a sterilized container that has been subjected to a sterilization process, and as illustrated in FIG. 1, includes an inlet portion 21 into which a preform P, as a container preform for a container such as a PET bottle, is introduced, a blow molding turret 22, an oven mechanism 30 that heats the preform P in an oven area A1 set downstream of the inlet portion 21, a preform conveyance mechanism 40 that conveys the preform P, and a container sterilization device 50 that sterilizes the preform P.

As illustrated in FIG. 1, the inlet portion 21 is a portion for introducing the preform P to the aseptic molding device 20 (aseptic filling system 10).

As illustrated in FIG. 1, the blow molding turret 22 is disposed downstream of the oven area A1 and performs aseptic blow-molding on the preform P.

Specifically, the blow molding turret 22 is configured as a turret including a blowing machine (not illustrated) that performs biaxial stretch blow-molding of a container such as a PET bottle by blowing aseptic air into the preform P that has been heated to a molding temperature at the time of aseptic blow-molding.

A molding area A3 in which the blow molding turret 22 is installed is covered by a chamber, and is kept in an aseptic atmosphere and at positive air pressure by aseptic air blown through a HEPA filter from above using a Fan Filter Unit (FFU).

Further, the blow molding turret 22, the blowing machine, and other equipment installed in the molding area A3 are sterilized up to the end of a blow pipe.

The preform P in which the container has been sterilized only by the sterilization process at the preform stage, is conveyed to the molding area A3, and is aseptically blow-molded.

As a result, aseptic blow-molding, in which blow molding is performed under the aseptic state, is performed in the molding area A3. Therefore, when conveying the preform P or the blow-molded container in the molding area A3, locally creating an atmosphere of aseptic air or sterilizing gas atmosphere in the vicinity of the conveying path is not needed, and further, sterilization of the blow-molded container is not needed.

The oven mechanism 30 performs a heating process to raise the temperature of (a body portion P1 of) the preform P to the molding temperature at the time of aseptic blow-molding in the blow molding turret 22, and includes an oven chamber 31 with the oven area A1 inside, and a plurality of temperature raising heaters 32 configured as infrared heaters that raise the temperature of the preform P.

The oven mechanism 30 may be provided with a simple cover surrounding the circumference of the oven instead of the oven chamber 31, or the oven chamber 31 may not be provided.

The body portion P1 of the preform P is a portion that is disposed inside a mold during aseptic blow-molding and is expanded within the mold, and a mouth portion P2 of the preform P is a portion that is disposed outside the mold during aseptic blow-molding and is not expanded.

As illustrated in FIG. 1, a transfer area A2 is an area downstream of the oven area A1 and upstream of the molding area A3, and is an area having a higher degree of contamination than the molding area A3 but having a lower degree of contamination than the oven area A1.

The preform conveyance mechanism 40 conveys the preform P inserted through the inlet portion 21 to the blow molding turret 22, and as illustrated in FIG. 1, includes a conveyor 41 that conveys the preform P mainly in the oven area A1, and a plurality of turrets 42 that convey the preform P unloaded from the oven area A1 toward the blow molding turret 22.

The turrets 42 convey the preform P in the transfer area A2 and the molding area A3, and the turrets 42 have a plurality of grippers that grip the preform P from the outer circumferential side. It is desirable that a gripping portion of the gripper is sterilized by UV light irradiation or a sterilizing agent.

The preform P may be conveyed by the preform conveyance mechanism 40 in any posture, such as an upright posture, an inverted posture (that is, a downward state in which the mouth portion P2 side of the preform P faces downward), or a horizontal posture (that is, a horizontal state in which the mouth portion P2 side of the preform P faces sideways).

The aseptic molding device 20 is configured to perform a sterilization process using the container sterilization device 50 or the like, such that the container is in a commercially aseptic state (that is, all the public health harmful microorganisms that can develop in the beverage under normal non-refrigerated storage and distribution conditions are killed) when the filling device 60 fills the content.

In the present embodiment, the sterilization process by the container sterilization device 50 and the like is configured such that sterilization of the container is completed only by the preform sterilization process at the preform stage before the container is molded by aseptic blow-molding without performing the sterilization process in the container stage after the aseptic blow-molding (no sterilization process is required at the container stage) (more specifically, in the present embodiment, sterilization is completed by the time of transfer area A2, and the sterilized preform P is transported to molding area A3). In other words, the sterilization process by the container sterilization device 50 and the like is configured such that the container is made commercially sterile only by the preform sterilization process at the preform stage, and in further other words, is configured such that the sterilizing target microorganisms (bacteria that may grow within the contents filled into the container, such as mold, general bacteria, spore-forming bacteria, and the like) can be sterilized (on the entire external surface including the inner and outer surfaces of the preform P) only by the preform sterilization process at the preform stage.

The sterilization effect (sterilization capability, D) is represented by the formula of sterilization effect (D) = LOG ((initial number of bacteria)/(number of survived bacteria)), and for example, when the number of bacteria is reduced from 100 to 10, LOG (100/10) = 1D.

The container sterilization device 50 is configured to sterilize the inner surface (the inner surface of the body portion P1 and the mouth portion P2) and the outer surface (the outer surface of the body portion P1 and the mouth portion P2) of the preform P in an area (in the present embodiment, the transfer area A2) downstream of the oven area A1 and upstream of the blow molding turret 22, and includes, as illustrated in FIG. 1, a laser irradiator 51 as a laser irradiation means that irradiates the container surface of the container or the preform P with a CO₂ laser, a UV light irradiator 52 as a UV light irradiation means that irradiates the container surface with UV light, and a heating means (in the present embodiment, the oven mechanism 30) disposed upstream of or at the same position as the laser irradiator 51 and configured to heat the region to be sterilized of the container surface.

The CO₂ laser is a kind of gas laser, and a gas obtained by mixing nitrogen and helium with carbon dioxide is used for the CO₂ (carbon dioxide gas) laser. By passing a discharge current through this mixed gas, a laser is extracted.

As illustrated in FIG. 1, the laser irradiator 51 irradiates the container surface of the preform P with a CO₂ laser in an area downstream of the oven area A1 and upstream of the blow molding turret 22 (in the present embodiment, the transfer area A2), while the preform P is being conveyed by the turret 42, thereby sterilizing the container surface of the preform P.

As illustrated in FIG. 2, the laser irradiator 51 includes a laser oscillator 51a and an irradiation unit 51b including optical elements such as a lens, a mirror, a beam expander, and a beam shaper, for emitting the CO₂ laser oscillated by the laser oscillator 51a.

The laser irradiator 51 (and the UV light irradiator 52) may be installed in the oven area A1 or upstream of the oven area A1. Further, the laser irradiator 51 (and the UV light irradiator 52) may be installed downstream of the blow molding turret 22, and the molded container may be irradiated with the CO₂ laser, thereby performing sterilization.

As illustrated in FIG. 1, in the area downstream of the oven area A1 and upstream of the blow molding turret 22 (in the present embodiment, the transfer area A2), the UV light irradiator 52 supplies sterilizing fluid to the container surface of the preform P while the preform P is being conveyed by the turret 42, thereby sterilizing the container surface of the preform P.

Here, the "UV light" referred to in the present specification includes ordinary UV light using a light source (UV light source 52a) such as a UV lamp (including a low-pressure mercury lamp), a xenon lamp (a xenon flash lamp, a xenon arc lamp), and a UV-LED, and a UV laser such as UV laser light generated by converting a YAG (Nd: YAG) laser, which is a solid-state laser, to a UV wavelength using wavelength-conversion technology, and UV laser light generated using an excimer laser, which is a gas laser, as a light source, but is not limited thereto.

As illustrated in FIG. 2, the UV light irradiator 52 includes the irradiation unit 51b including optical elements such as a lens, a mirror, a beam expander, and a beam shaper, for emitting the UV light emitted from the UV light source 52a. The irradiation unit 51b is not essential, and the UV light may be directly emitted from the UV light source 52a.

The heating means is performed before or simultaneously with the laser irradiation process by the laser irradiator 51 (and the UV light irradiation process by the UV light irradiator 52), and heats the region to be sterilized such that the region to be sterilized on the container surface is heated during the laser irradiation process (and the UV light irradiation process). In the present embodiment, the oven mechanism 30 described above functions as the heating means.

The specific aspect of the heating means is not limited to the above, and the heating means may be constituted by a temperature raising heater provided separately from the oven mechanism 30, a supplier or the like that supplies hot water, water vapor, hot air, or the like. In this case, the heating temperature of the region to be sterilized can be appropriately set to 60°C or more, 80°C or more, 100°C or more, or the like.

The filling device 60 is installed downstream of the aseptic molding device 20, and as illustrated in FIG. 1, includes a filling unit 61 as a filling means that fills the container with the content, and a capping unit 62 that is disposed downstream of the filling unit 61 and attaches a sterilized cap to the mouth portion of the container.

Each step in the filling device 60 is performed in a chamber whose inside is maintained in an aseptic state.

In addition, the aseptic state of the container is maintained also in the section in which the container is conveyed between the aseptic molding device 20 and the filling device 60. In other words, the aseptic state of the container is maintained in the entire section from after the container is aseptically blow-molded by the blow molding turret 22 to before the container is aseptically filled with the content in the filling unit 61, and in the present embodiment, by covering the entire section with the chamber, the aseptic state of the container is maintained in the section.

Next, a container sterilization method using the container sterilization device 50 of the present embodiment will be described below.

First, the container sterilization method of the present embodiment includes a heating process to be performed before or simultaneously with the laser irradiation process, the laser irradiation process of irradiating the container surface with a CO₂ laser, and the UV light irradiation process of irradiating the container surface with UV light.

The laser irradiation process is to irradiate the container surface (in the present embodiment, the entire surface including the inner surface and the outer surface of the preform P) with a CO₂ laser by the laser irradiator 51, and is performed on a path, of aseptic filling equipment, between the inlet portion 21 and the filling unit 61 where a filling process is performed.

The UV light irradiation process is to irradiate the container surface (in the present embodiment, the entire surface including the inner surface and the outer surface of the preform P) with a CO₂ laser by the UV light irradiator 52, and is performed on a path, of the aseptic filling equipment, between the inlet portion 21 and the filling unit 61 where the filling process is performed.

In the UV light irradiation process, a UV laser is preferably used for the irradiation.

As illustrated in FIG. 2, it is preferable that an irradiated region of the CO₂ laser on the container surface in the laser irradiation process and an irradiated region of the UV light on the container surface in the UV light irradiation process are set to (at least partially) overlap each other.

As a specific aspect of the laser irradiation process and the UV light irradiation process, as illustrated in FIG. 2(a), the irradiation unit 51b of the laser irradiator 51 and the irradiation unit 52b of the UV light irradiator 52 may be separately provided, and the respective irradiated regions may be overlapped. Alternatively, as illustrated in FIG. 2(b), by mixing the CO₂ laser and the UV laser and emitting the mixed laser from the same irradiation unit 51b and 52b, the respective irradiated regions may be overlapped. Furthermore, the UV light may be directly emitted from the UV light source 52a without using the irradiation unit 52b of the UV light irradiator 52.

Here, reference numeral 53 illustrated in FIG. 2 denotes a mixer that mixes the CO₂ laser and the UV laser, reference numeral Lc illustrated in FIG. 2 denotes the CO₂ laser, and reference numeral Lu illustrated in FIG. 2 denotes the UV laser.

In the example illustrated in FIG. 2, the CO₂ laser irradiation and the UV light irradiation process are simultaneously performed. However, the CO₂ laser irradiation and the UV light irradiation process may be performed at different timings (the CO₂ laser irradiation is performed before or after the UV light irradiation process).

Further, it is preferable to set the laser irradiation process and the UV light irradiation process performed with respect to the preform after the heating process such that the integrated light amount (mJ/cm²) of the CO₂ laser to the mouth portion P2 of the preform is greater than the integrated light amount (mJ/cm²) of the CO₂ laser to the body portion P1 of the preform, and/or the integrated light amount (mJ/cm²) of the UV light to the mouth portion P2 of the preform is greater than the integrated light amount (mJ/cm²) of the UV light to the body portion P1 of the preform (for both the inner and outer surfaces of the preform P or only the inner or outer surfaces of the preform P).

In the laser irradiation process, the CO₂ laser having a wavelength in the band of 9 µm (center wavelength of 9.0 to 9.9 µm) is preferably used for the irradiation.

In the laser irradiation process, it is preferable to emit the CO₂ laser for an irradiation time of 1 second or less. This can significantly shorten the sterilization time of the container. The irradiation time here means the time during which the region to be sterilized receives the CO₂ laser light.

In addition, in the laser irradiation process, in order to obtain a good sterilization effect while avoiding damage to the container surface to be irradiated, the CO₂ laser is preferably emitted such that the integrated light amount of the CO₂ laser in the region to be sterilized on the container surface is 1600 mJ/cm² or more and 30000 mJ/cm² or less.

In addition, in the laser irradiation process, the CO₂ laser may be emitted in either a continuous wave or pulse wave mode.

The heating process is to heat the region to be sterilized on the container surface by the laser irradiation process using the heating means, and in the present embodiment, the preform P is heated to the molding temperature at the time of the container molding process by the oven mechanism 30 as the heating means.

The heating process is preferably set to heat the region to be sterilized such that the temperature of the region to be sterilized on the container surface becomes 120°C or more during the laser irradiation process performed on the preform P after the heating process.

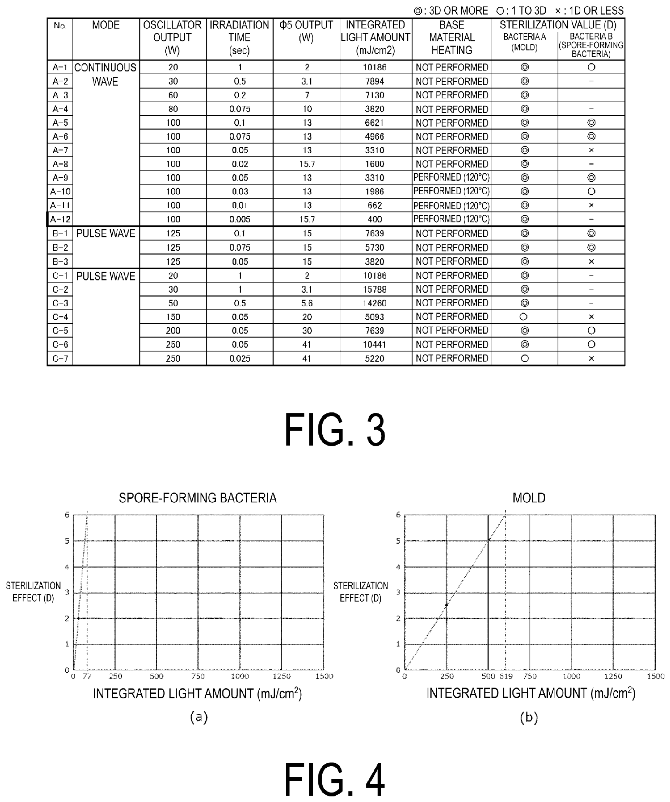

Next, a test conducted to confirm the sterilization effect by the CO₂ laser irradiation will be described with reference to FIG. 3.

In this test, first, 1.7 × 10³ cfu of spore-forming bacteria (Bacillus atrophaeus spores) and 1.2 × 10³ cfu of mold (Aspergillus niger conidia) were attached to a circular region with a diameter of 5 mm on a plate-shaped base material made of polyethylene terephthalate (PET), and then dried in a clean room to prepare a base material inoculated with spore-forming bacteria and mold.

Then, the prepared base material was irradiated with a CO₂ laser under the conditions illustrated in FIG. 3, and then the number of survived bacteria on the base material was measured by the following standard method.

Method for measuring the number of survived bacteria: The above-described base material, sterilized aqueous surfactant solution, and sterilized beads were placed in a sterilized test tube, the test tube was then sealed with a sterilized cap. The test tube was shaken to extract the survived bacteria in the aqueous surfactant solution, which were then suspended in a sterilized dilution solution. The number of bacteria was measured by a membrane filter method on a standard agar medium at each dilution ratio. The medium was cultured at 35°C for one week, and the number of colonies that appeared according to the dilution ratio was counted to determine the number of survived bacteria.

The diameter of the CO₂ laser irradiation spot on the base material surface was approximately 22 mm, and the "Φ5 output (W)" illustrated in FIG. 3 was, out of the output (W) of the laser oscillator during the test, the measured output (W) that was emitted onto a circular region with a diameter of 5 mm where the bacteria had been inoculated.

In addition, the base materials of A-9 to 12 were heated by a heater such that the region to be irradiated of the base material reached 120°C when irradiated with the CO₂ laser.

In addition, in A-1 to 12, the CO₂ laser used for irradiation was a continuous wave, and the output was adjusted by PWM control (frequency 5 kHz). In B-1 to 3 and C-1 to 7, the CO₂ laser used for irradiation was a pulse wave and the frequency was 1 kHz.

Further, the wavelength of the CO₂ laser used for irradiation in A-1 to 12 and B-1 to 3 was in the band of 9 µm (9.3 µm), and the wavelength of the CO₂ laser used for irradiation in C-1 to 7 was in the band of 10 µm (10.6 µm).

In addition, in this test, the test was conducted by configuring an optical system using a laser oscillator, an irradiation lens as the irradiation unit, and the like. Further, the "Φ5 output (W)" was measured using a power meter (UP55N-300F-H12-D0) manufactured by Gentec Corporation.

The following was found from the results of the above tests.

That is, it was found that, in the case where the wavelength of the CO₂ laser was in the band of 9 µm, a good sterilization effect on mold was obtained when the irradiation time was in the range of 0.02 seconds or more and 0.05 seconds or less, and 1600 mJ/cm² or more (more preferably, 3310 mJ/cm² or more).

Further, it was found that, in the case where the wavelength of the CO₂ laser was in the band of 9 µm, a good sterilization effect on mold was obtained when the irradiation time was in the range of 0.05 seconds or more and 0.075 seconds or less, and 3310 mJ/cm² or more (more preferably, 3820 mJ/cm² or more).

Further, it was found that, in the case where the wavelength of the CO₂ laser was in the band of 9 µm, a good sterilization effect on mold was obtained when the irradiation time was in the range of more than 0.075 seconds and 0.1 seconds or less, and 3820 mJ/cm² or more (more preferably, 6621 mJ/cm² or more).

Further, it was found that, in the case where the wavelength of the CO₂ laser was in the band of 9 µm, a good sterilization effect on mold was obtained when the irradiation time was in the range of more than 0.1 seconds and 0.2 seconds or less, and 6621 mJ/cm² or more (more preferably, 7130 mJ/cm² or more).

Further, it was found that, in the case where the wavelength of the CO₂ laser was in the band of 9 µm, a good sterilization effect on mold was obtained when the irradiation time was in the range of more than 0.2 seconds and 0.5 seconds or less, and the 7130 mJ/cm² or more (more preferably, 7894 mJ/cm² or more).

Further, it was found that, in the case where the wavelength of the CO₂ laser was in the band of 9 µm, a good sterilization effect on mold was obtained when the irradiation time was in the range of more than 0.5 seconds and 1.0 seconds or less, and 7894 mJ/cm² or more (more preferably, 10186 mJ/cm² or more).

Further, it was found that, in the case where the wavelength of the CO₂ laser was in the band of 10 µm, a good sterilization effect on mold was obtained when the irradiation time was in the range of 0.05 seconds or more and 1.0 seconds or less, and 5093 mJ/cm² or more (more preferably, 10200 mJ/cm² or more).

Further, it was found that, in the case where the wavelength of the CO₂ laser was in the band of 9 µm and the base material heating was performed, a good sterilization effect on mold was obtained when the irradiation time was in the range of 0.005 seconds or more and 0.01 seconds or less, and 400 mJ/cm² or more (more preferably, 662 mJ/cm² or more).

Further, it was found that, in the case where the wavelength of the CO₂ laser was in the band of 9 µm and the base material heating was performed, a good sterilization effect on mold was obtained when the irradiation time was in the range of 0.01 seconds or more and 0.03 seconds or less, and 662 mJ/cm² or more (more preferably, 1986 mJ/cm² or more).

Further, it was found that, in the case where the wavelength of the CO₂ laser was in the band of 9 µm and the base material heating was performed, a good sterilization effect on mold was obtained when the irradiation time was in the range of 0.03 seconds or more and 0.05 seconds or less, and 1986 mJ/cm² or more (more preferably, 3310 mJ/cm² or more).

Therefore, it was found that even when the irradiation time was set to 1 second or less (0.025 seconds or more when the base material heating was not performed, and 0.005 seconds or more when the base material heating was performed), the mold could be sterilized by appropriately setting the output of the CO₂ laser.

Further, it was found that, in the case where the wavelength of the CO₂ laser was in the band of 9 µm, a good sterilization effect on the spore-forming bacteria was obtained when the irradiation time was in the range of 0.05 seconds or more and 0.075 seconds or less, and the 4966 mJ/cm² or more.

Further, it was found that, in the case where the wavelength of the CO₂ laser was in the band of 9 µm, a good sterilization effect on the spore-forming bacteria was obtained when the irradiation time was in the range of more than 0.075 seconds and 0.1 seconds or less, and 4966 mJ/cm² or more (more preferably, 6621 mJ/cm² or more).

Further, it was found that, in the case where the wavelength of the CO₂ laser was in the band of 9 µm, a good sterilization effect on the spore-forming bacteria was obtained when the irradiation time was in the range of more than 0.1 seconds and 1.0 seconds or less, and 6621 mJ/cm² or more (more preferably, 10186 mJ/cm² or more).

Further, it was found that, in the case where the wavelength of the CO₂ laser was in the band of 10 µm, a good sterilization effect on the spore-forming bacteria was obtained when the irradiation time was in the range of 0.05 seconds or more and 1.0 seconds or less, and 7639 mJ/cm² or more.

Therefore, it was found that even when the irradiation time was set to 1 second or less (0.05 seconds or more), the spore-forming bacteria could be sterilized by appropriately setting the output of the CO₂ laser.

In addition, from the comparison between A-7 and A-9, it was found that the sterilization effect on spore-forming bacteria was improved when the base material was heated.

It was also found that the sterilization effect was not significantly affected by whether the continuous wave or the pulse wave was used.

It was also found that the higher the output of the CO₂ laser (Φ5 output) and the shorter the irradiation time, the smaller the integrated light amount required for the sterilization.

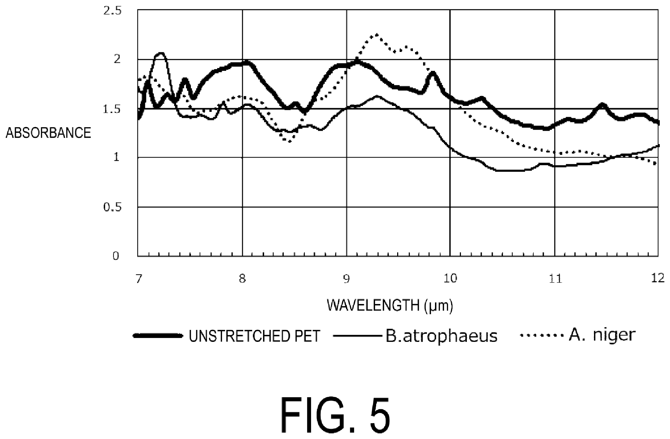

The mechanism by which the CO₂ laser exhibits the excellent sterilization effect as described above is not necessarily clear. However, according to the absorbances of the spore-forming bacteria (Bacillus atrophaeus), the mold (Aspergillus niger), and the PET resin with respect to the light wavelength shown in FIG. 5, the spore-forming bacteria, the mold, and the PET resin all exhibit high absorbances in the wavelength band of the CO₂ laser, particularly in the band of 9 µm. The bacteria absorb the CO₂ laser light and are directly heated, causing DNA destruction, and the PET resin is also heated by the laser light to indirectly heat the bacteria, which is thought to provide an excellent sterilization effect. The absorbance was measured by the KBr pellet method using a Fourier transform infrared spectrophotometer (FT/IR6700, manufactured by JASCO Corporation).

In the present embodiment obtained in this manner, by using both the laser irradiation process of irradiating the container surface with the CO₂ laser and the UV light irradiation process of irradiating the container surface with the UV light, both the spore-forming bacteria and the mold can be favorably sterilized with a small output and in a short time, and the device configuration of each irradiator can be simplified, as compared with a case where both the spore-forming bacteria and the mold are sterilized only by the UV light irradiation or only by the CO₂ laser irradiation.

That is, if it is attempted to obtain a sterilization effect equivalent to 6D for both spore-forming bacteria and mold only by UV light irradiation, as can be seen from the graph shown in FIG. 4, UV light (UV laser) irradiation of 77 mJ/cm² is sufficient to sterilize the spore-forming bacteria, but UV light (UV laser) irradiation of 619 mJ/cm² is required to sterilize the mold. Here, if the CO₂ laser irradiation is used in combination and the mold is sterilized by the CO₂ laser irradiation, the UV light irradiation of 77 mJ/cm² is sufficient.

The graph shown in FIG. 4(a) is an estimation of the sterilization effect by the UV laser based on the integrated light amount value (value obtained in the test) of the UV laser required to obtain the sterilization effect of 2.0D for the spore-forming bacteria, and the graph shown in FIG. 4(b) is an estimation of the sterilization effect by the UV laser based on the integrated light amount value (value obtained in the test) of the UV laser required to obtain the sterilization effect of the 2.5D for the mold.

Further, in the test described above, 1.4 × 10² cfu of spore-forming bacteria (Bacillus atrophaeus spores) and 4.3 × 10³ cfu of mold (Aspergillus niger conidia) were attached to a plate-shaped base material made of polyethylene terephthalate (PET), and then dried in a clean room to prepare a base material inoculated with the spore-forming bacteria and mold. The prepared base material was irradiated with a UV laser having a center wavelength of 266 nm, and then the number of survived bacteria on the base material was measured using the standard method described above.

From the graph shown in FIG. 4, it can be seen that, to achieve a sterilization effect equivalent to 6D for spore-forming bacteria by UV laser irradiation, it is estimated that UV light (UV laser) irradiation of 77 mJ/cm² is required, and to achieve a sterilization effect equivalent to 6D for mold by UV laser irradiation, it is estimated that UV light (UV laser) irradiation of 619 mJ/cm² is required.

As can be seen from the sterilization effects shown in FIGS. 3 and 4, since the UV light (UV laser) irradiation tends to be excellent in sterilization of spore-forming bacteria and the CO₂ laser irradiation tends to be excellent in sterilization of mold, the laser irradiation process and the UV light irradiation process are preferably set such that the sterilization capability (D) against mold by the laser irradiation process is higher than the sterilization capability (D) against mold by the UV light irradiation process from the viewpoint of suppressing the total output.

Further, from the same viewpoint, the laser irradiation process and the UV light irradiation process are preferably set such that the sterilization capability (D) against spore-forming bacteria by the laser irradiation process is lower than the sterilization capability (D) against spore-forming bacteria by the UV light irradiation process.

Furthermore, by adopting the sterilization by CO₂ laser irradiation and the UV light irradiation, sterilization using a sterilizing agent is not required, or even if sterilization by a sterilizing agent is used in combination, the amount of sterilizing agent used can be reduced, thereby reducing the risk of the sterilizing agent remaining in the molded container.

Further, the heating process performed before or at the same time as the laser irradiation process is included, and in this heating process, by heating the region to be sterilized on the container surface by the laser irradiation process, the sterilization effect of the region to be sterilized on the container surface can be further improved.

The embodiment of the present invention has been described in detail above, but the present invention is not limited to the embodiment described above, and various design modifications can be made insofar as they do not depart from the scope of the claims of the present invention. Furthermore, the container sterilization method and the container sterilization device 50 may be configured by any combination of each configuration of the above-described embodiment and modified examples to be described below.

In the above-described embodiment, the container sterilization method (container sterilization device 50) has been described as being configured to sterilize the container surface of the preform P which is a container preform. However, a specific aspect of the aseptic filling method (aseptic filling system 10) is not limited to the above, and the container surface of the container after the molding may be sterilized.

In addition, in the above-described embodiment, the container preform is the preform P and the container is the PET bottle. However, the specific aspects of the container preform and the container are not limited thereto. For example, the container may be a resin-coated can, a painted can, or the like. In addition, the container preform may be a sheet material (for example, a sheet material in which the surface of a paper base material is coated with a synthetic resin, or a sheet material formed as a resin film), and the container may be a sheet material container (for example, a paper pack or a pouch) obtained by molding the sheet material.

In this case as well, the laser irradiation process and the UV light irradiation process are performed with respect to the sheet material or the like as the container preform, or the sheet material container or the like as the container.

Further, in this case, in the laser irradiation process and the UV light irradiation process, the irradiation spot of the CO₂ laser and the UV light may be set in a horizontally long shape with a horizontal width in a direction orthogonal to a conveying direction of the sheet material serving as the container preform wider than a vertical width in the conveying direction, and the sheet material may be irradiated with the CO₂ laser and the UV light while the sheet material is conveyed.

Further, in the case where the container surface is formed of synthetic resins such as polyethylene terephthalate (PET), polypropylene (PP), or polyethylene (PE), (or coated with synthetic resins), in order to avoid damage to the container surface caused by the CO₂ laser, the container surface is preferably irradiated with the CO₂ laser such that the integrated light amount of the CO₂ laser on the region to be sterilized is 30000 mJ/cm² or less.

In addition, in the above-described embodiment, the container sterilization device 50 has been described as including the laser irradiator 51, the UV light irradiator 52, and the like. However, as a constituent element of the container sterilization device 50, a sterilization treatment device other than the laser irradiator 51, for example, a sterilization treatment device that performs sterilization by supplying sterilization fluid such as hydrogen peroxide (hydrogen peroxide aqueous solution), peracetic acid (peracetic acid aqueous solution), or water (hot water, water vapor) to the container surface of the container or the container preform, a sterilization treatment device that performs sterilization by irradiating the container surface with an electron beam, a dust remover that removes dust attached to the preform P, or the like may be provided. When water (hot water, water vapor) is used as the sterilization fluid, the risk of the sterilizing agent remaining in the container to be molded can be eliminated.

In addition, in the above-described embodiment, in the laser irradiation process, the CO₂ laser with a wavelength in the band of 9 µm (center wavelength of 9.0 to 9.9 µm, such as 9.3 µm or 9.6 µm) is used for the irradiation. However, the CO₂ laser with another wavelength, for example, a band of 10 µm (center wavelength of 10.0 to 10.9 µm, such as 10.2 µm or 10.6 µm) may be used for the irradiation.

Further, the wavelength of the UV light used for the irradiation in the UV light irradiation process is preferably set to have a center wavelength of 240 to 280 nm. In particular, when the UV laser is used for the irradiation, the wavelength of the UV laser is preferably set to the center wavelength of 266 nm (UV laser light generated by a YAG (Nd: YAG) laser using wavelength-conversion technology) or the center wavelength of 248 nm (UV laser light generated using an excimer laser as a UV light source). In addition, the center wavelength is preferably set to 254 nm in the case of a UV lamp (low-pressure mercury lamp), 240 to 280 nm in the case of a xenon flash lamp, and 265 nm in the case of a UV-LED.

Further, in the above-described embodiment, the CO₂ laser and the UV light are used for the irradiation with respect to the entire surface including the inner surface and the outer surface among the container surface of the container or the container preform. However, the CO₂ laser and the UV light may be used for the irradiation with respect to only a portion of the container surface (for example, only the inner surface, only the outer surface, only the inner surface of the mouth portion, or the like).

In addition, in the above-described embodiment, the container surface is sterilized by being irradiated with the CO₂ laser. However, instead of the CO₂ laser, the container surface may be sterilized by being irradiated with an infrared wavelength laser (specifically, a laser with a wavelength of 0.78 µm to 1000 µm including a laser with a near-infrared wavelength, a mid-infrared wavelength, and a far-infrared wavelength). As the type of laser, in addition to the CO₂ laser, a known laser such as a diode laser, a fiber laser, a YAG laser, a YVO₄ laser, or a CO laser may be used.

Compared to known infrared lamps and heaters, the infrared wavelength laser is advantageous in its high energy efficiency since it has high directionality and can intensively irradiate the sterilizing target portion with infrared rays, and also has the advantage of having a fast output rise when irradiation begins, allowing for short-term ON-OFF control.

The specific aspects of an aseptic filling method and system using an infrared wavelength laser are exactly the same as the aseptic filling method and system using the CO₂ laser described above, except that an infrared wavelength laser is used instead of the CO₂ laser. Therefore, by replacing "CO₂ laser" in the explanation of the aseptic filling method and system using the CO₂ laser described above with "laser with an infrared wavelength", the specific description thereof will be omitted.

Furthermore, the above-described aseptic filling method and system using an infrared wavelength laser can also be described as in the following supplementary notes, but are not limited to the following.

### Supplementary Note 1

A container sterilization method for sterilizing a container surface of a container or a container preform, the container sterilization method including:
a laser irradiation process of irradiating the container surface with a laser having an infrared wavelength; and
a UV light irradiation process of irradiating the container surface with UV light.

### Supplementary Note 2

The container sterilization method according to Supplementary Note 1, in which an irradiated region of the laser having the infrared wavelength in the laser irradiation process and an irradiated region of the UV light in the UV light irradiation process overlap each other.

### Supplementary Note 3

The container sterilization method according to Supplementary Note 1 or 2, in which in the UV light irradiation process, a UV laser is used for irradiation.

### Supplementary Note 4

The container sterilization method according to Supplementary Note 3, in which the laser irradiation process and the UV light irradiation process are performed by mixing the laser having the infrared wavelength and the UV laser and emitting the mixed laser from an identical irradiation unit.

### Supplementary Note 5

The container sterilization method according to any one of Supplementary Notes 1 to 4, in which a sterilization capability against mold by the laser irradiation process is set to be higher than a sterilization capability against mold by the UV light irradiation process.

### Supplementary Note 6

The container sterilization method according to any one of Supplementary Notes 1 to 5, in which a sterilization capability against spore-forming bacteria by the laser irradiation process is set to be lower than a sterilization capability against spore-forming bacteria by the UV light irradiation process.

### Supplementary Note 7

The container sterilization method according to any one of Supplementary Notes 1 to 6, further including:
a heating process to be performed before or simultaneously with the laser irradiation process, in which
in the heating process, a region to be sterilized on the container surface by the laser irradiation process is heated.

### Supplementary Note 8

The container sterilization method according to any one of Supplementary Notes 1 to 7, further including:
a heating process of heating a preform as the container preform to a molding temperature at which the preform is molded into a PET bottle as the container, in which
the laser irradiation process and the UV light irradiation process are performed with respect to the preform after the heating process, and
an integrated light amount (mJ/cm²) of the laser having the infrared wavelength to a mouth portion of the preform is greater than an integrated light amount (mJ/cm²) of the laser having the infrared wavelength to a body portion of the preform, and/or an integrated light amount (mJ/cm²) of the UV light to the mouth portion of the preform is greater than an integrated light amount (mJ/cm²) of the UV light to the body portion of the preform.

### Supplementary Note 9

A container sterilization device for sterilizing a container surface of a container or a container preform, including:
a laser irradiation means configured to irradiate the container surface with a laser having an infrared wavelength; and
a UV light irradiation means configured to irradiate the container surface with UV light.

### Reference Signs List

10 Aseptic filling system
20 Aseptic molding device
21 Inlet portion
22 Blow molding turret
30 Oven mechanism
31 Oven chamber
32 Temperature raising heater
40 Preform conveyance mechanism
41 Conveyor
42 Turret
50 Container sterilization device
51 Laser irradiator (laser irradiation means)
51a Laser oscillator
51b Irradiation unit
52 UV light irradiator (UV light irradiation means)
52a UV light source
52b Irradiation unit
53 Mixer
60 Filling device
61 Filling unit
62 Capping unit
P Preform (container preform)
P1 Body portion of preform
P2 Mouth portion of preform
A1 Oven area
A2 Transfer area
A3 Molding area
Lc CO₂ laser
Lu UV light

## Claims

1. A container sterilization method for sterilizing a container surface of a container or a container preform, the container sterilization method comprising:
a laser irradiation process of irradiating the container surface with a CO₂ laser; and
a UV light irradiation process of irradiating the container surface with UV light.

2. The container sterilization method according to claim 1, wherein an irradiated region of the CO₂ laser in the laser irradiation process and an irradiated region of the UV light in the UV light irradiation process overlap each other.

3. The container sterilization method according to claim 1, wherein in the UV light irradiation process, a UV laser is used for irradiation.

4. The container sterilization method according to claim 3, wherein the laser irradiation process and the UV light irradiation process are performed by mixing the CO₂ laser and the UV laser and emitting the mixed laser from an identical irradiation unit.

5. The container sterilization method according to claim 1, wherein a sterilization capability against mold by the laser irradiation process is set to be higher than a sterilization capability against mold by the UV light irradiation process.

6. The container sterilization method according to claim 1, wherein a sterilization capability against spore-forming bacteria by the laser irradiation process is set to be lower than a sterilization capability against spore-forming bacteria by the UV light irradiation process.

7. The container sterilization method according to claim 1, further comprising:
a heating process to be performed before or simultaneously with the laser irradiation process, wherein
in the heating process, a region to be sterilized on the container surface by the laser irradiation process is heated.

8. The container sterilization method according to claim 1, further comprising:
a heating process of heating a preform as the container preform to a molding temperature at which the preform is molded into a PET bottle as the container, wherein
the laser irradiation process and the UV light irradiation process are performed with respect to the preform after the heating process, and
an integrated light amount (mJ/cm²) of the CO₂ laser to a mouth portion of the preform is greater than an integrated light amount (mJ/cm²) of the CO₂ laser to a body portion of the preform, and/or an integrated light amount (mJ/cm²) of the UV light to the mouth portion of the preform is greater than an integrated light amount (mJ/cm²) of the UV light to the body portion of the preform.

9. A container sterilization device for sterilizing a container surface of a container or a container preform, comprising:
a laser irradiation means configured to irradiate the container surface with a CO₂ laser; and
a UV light irradiation means configured to irradiate the container surface with UV light.

10. A container sterilization method for sterilizing a container surface of a container or a container preform, the container sterilization method comprising:
a laser irradiation process of irradiating the container surface with a laser having an infrared wavelength; and
a UV light irradiation process of irradiating the container surface with UV light.

11. A container sterilization device for sterilizing a container surface of a container or a container preform, comprising:
a laser irradiation means configured to irradiate the container surface with a laser having an infrared wavelength; and
a UV light irradiation means configured to irradiate the container surface with UV light.
